# EUROPEAN PATENT APPLICATION

(11) **EP 4 624 926 A1**
(43) Date of publication of application: **01.10.2025**
(21) Application number: 25165874.6
(22) Date of filing: 25.03.2025
(51) Int. Cl.: G01N 33/50, G01N 33/58, G01N 33/569

(54) **A METHOD FOR ANALYZING A URINE SAMPLE, AND A REAGENT FOR ANALYZING A URINE SAMPLE**

(30) Priority: 27.03.2024 JP 2024051107
(71) Applicant: SYSMEX CORPORATION, Kobe-shi Hyogo 651-0073 (JP)
(72) Inventor: TAMURA, Tomoki, Kobe-shi, Hyogo 651-0073 (JP); YAMAZAKI, Moe, Kobe-shi, Hyogo 651-0073 (JP); KONISHI, Ikumi, Kobe-shi, Hyogo 651-0073 (JP)
(74) Representative: Hoffmann Eitle

(57) **Abstract**

[Problem] To provide a method for analyzing a urine sample capable of discriminating red blood cells in a specimen with higher accuracy, and a reagent for analyzing a urine sample.

[Solution] obtaining a measurement sample comprising: the urine sample; at least one compound selected from the group consisting of aliphatic compound and aromatic compound; and a fluorescent dye that stains nucleic acids or cytoplasm; irradiating the measurement sample with light and obtaining fluorescence information of particles contained in the measurement sample; and detecting red blood cells based on the fluorescence information, wherein the aliphatic compound is at least one selected from the group consisting of 1,2-alkanediol having 8 to 10 carbon atoms and 2-alkanol having 7 to 9 carbon atoms, and the aromatic compound is chlorphenesin, 3-phenoxy-1-propanol, 1-(3-methoxyphenoxy)-2-propanol A method for analyzing a urine sample, wherein the urine sample is at least one selected from the group consisting of orthophenylphenol and orthophenylphenol.

## Description

### [Technical field]

The present invention relates to a method for analyzing a urine sample and a reagent for analyzing a urine sample.

### [Background of the Invention]

Analyzing red blood cells that appear in the urine is important in determining the presence or absence of bleeding in the pathway from the glomeruli of the kidney to the urethra. Further, a bleeding site can be identified from the morphological information of red blood cells, and is important information at the time of diagnosis.

Patent Document 1 describes a method for analyzing urinary components such as red blood cells by flow cytometry. Patent Document 1 discloses that a fraction with yeast-like fungi may not be good when analyzing red blood cells, and that a fraction with red blood cells and yeast-like fungi can be improved by a predetermined compound.

### [Prior art reference]

### [Patent document]

[Patent document 1] U.S. Patent Application Publication No. 2006/0073601

### [Summary of the invention]

### [Problem to be solved by the invention]

However, it is desired to be able to discriminate red blood cells with higher accuracy.

An object of the present invention is to provide a method for analyzing a urine sample capable of discriminating red blood cells in a specimen with higher accuracy, and a reagent for analyzing a urine sample.

### [Means for solving the problem]

As a result of intensive studies by the present inventors, it has been found that red blood cells in a specimen can be discriminated with high accuracy by including at least one compound selected from the group consisting of specific aliphatic compounds and specific aromatic compounds in a measurement sample, and thus the present invention has been completed.

The present invention provides a method for analyzing a urine sample, comprising:
- obtaining a measurement sample comprising: the urine sample; at least one compound selected from the group consisting of aliphatic compounds and aromatic compounds; and a fluorescent dye that stains nucleic acids or cytoplasm;
- irradiating the measurement sample with light and obtaining fluorescence information of particles contained in the measurement sample; and
- detecting red blood cells based on the fluorescence information,

wherein the aliphatic compound is at least one selected from the group consisting of 1,2-alkanediol having 8 to 10 carbon atoms and 2-alkanol having 7 to 9 carbon atoms, and
wherein the aromatic compound is at least one selected from the group consisting of chlorphenesin, 3-phenoxy-1-propanol, 1-(3-methoxyphenoxy)-2-propanol, and orthophenylphenol.

The present invention provides a method for analyzing a urine sample, comprising:
- obtaining a measurement sample comprising: the urine sample; at least one compound selected from the group consisting of aliphatic compound and aromatic compound; and a fluorescent dye that stains nucleic acids or cytoplasm;
- irradiating the measurement sample with light and obtaining fluorescence information of particles contained in the measurement sample; and
- discriminating between red blood cells and yeast-like fungi based on the fluorescence information;

wherein the aliphatic compound is at least one selected from the group consisting of 1,2-alkanediol having 8 to 10 carbon atoms and 2-alkanol having 7 to 9 carbon atoms, and
wherein the aromatic compound is at least one selected from the group consisting of chlorphenesin, 3-phenoxy-1-propanol, 1-(3-methoxyphenoxy)-2-propanol, and orthophenylphenol.

### [Effect of the invention]

According to the present invention, it is possible to provide a method for analyzing a urine sample capable of discriminating red blood cells in a specimen with higher accuracy, and a reagent for analyzing a urine sample.

### [Brief Description of the Drawings]

FIG. 1 is a flowchart illustrating an example of a urine sample analysis method according to a first embodiment.
FIG. 2 is a flowchart illustrating an example of a urine sample analysis method according to the second embodiment.
FIG. 3 is a scattergram obtained in Example 1.
FIG. 4 is a scattergram obtained in Example 1.
FIG. 5 is a scattergram obtained in Example 2.
FIG. 6 is a scattergram obtained in Example 3.
FIG. 7 is a scattergram obtained in Example 4.
FIG. 8 is a scattergram obtained in Example 4.

### [DETAILED DESCRIPTION OF THE PREFERRED EMBODIMENTS]

Hereinafter, an embodiment of the present invention (hereinafter, referred to as "the present embodiment") will be described in detail. However, the present invention is not limited to the following embodiments, and various modifications can be made without departing from the gist thereof.

### [urine sample analysis method]

In the method for analyzing a urine sample according to the present embodiment, the measurement sample includes at least one compound selected from the group consisting of a specific aliphatic compound and a specific aromatic compound, so that red blood cells in the specimen can be discriminated with high accuracy. In particular, in the analysis of the urine sample, red blood cells and yeast-like fungi can be sufficiently fractionated, and red blood cells in the specimen can be measured with high accuracy.

### (The First Embodiment)

A method for analyzing a urine sample according to the first embodiment includes the steps of obtaining a measurement sample comprising: the urine sample; at least one compound selected from the group consisting of aliphatic compound and aromatic compound (hereinafter, also referred to as "compound of the present embodiment"); and a fluorescent dye that stains nucleic acids or cytoplasm; irradiating the measurement sample with light and obtaining fluorescence information of particles contained in the measurement sample; and detecting red blood cells based on the fluorescence information.

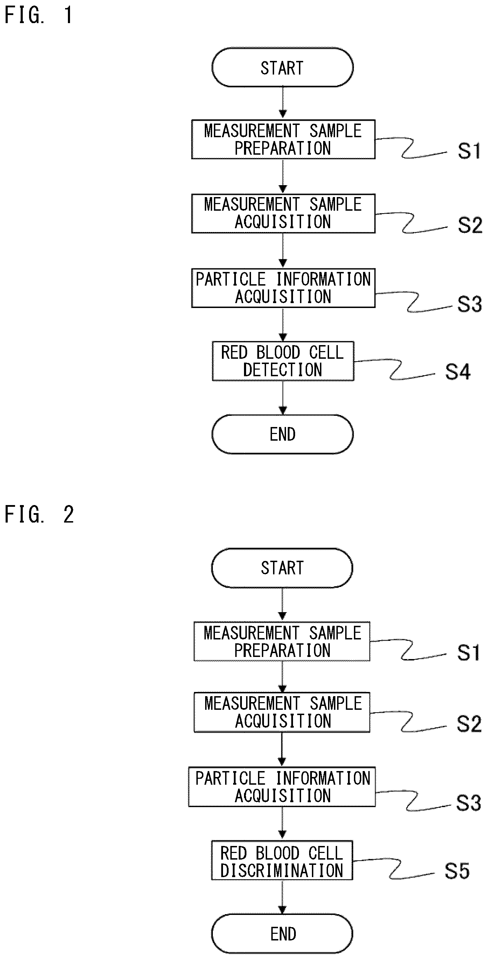

FIG. 1 is a flowchart illustrating an example of a method for analyzing a urine sample according to a first embodiment. As shown in FIG. 1, in the urine sample analysis method according to the first embodiment, the measurement sample is prepared (step S1), the measurement apparatus acquires the measurement sample (step S2), acquires particle information from the measurement sample (step S3), and detects red blood cells from the specimen based on the acquired particle information (step S4).

### (Measurement sample preparation step)

In the measurement sample preparation step S1, a measurement sample is prepared from a urine sample that is a specimen. In step S1, the measurement sample is prepared by mixing the urine sample with a first reagent comprising at least one compound selected from the group consisting of aliphatic compound and aromatic compound, and a second reagent comprising a fluorescent dye that stains nucleic acids or cytoplasm.

The urine sample is a sample comprising urine that is a specimen, preferably urine taken from a subject. In the case where urine collected from a subject is used as a sample, it is preferably used within 24 hours after collection in order to suppress a change in components in the urine sample due to a lapse of time.

The first reagent comprises a compound of this embodiment and is a reagent that adds the compound to a urine sample and dilutes the urine sample. In the present embodiment, since the measurement sample includes, in addition to the urine sample, at least one compound selected from the group consisting of a specific aliphatic compound and a specific aromatic compound, red blood cells in the specimen can be discriminated with high accuracy.

The aliphatic compound in the compound of the present embodiment is at least one selected from the group consisting of 1,2-alkanediol having 8 to 10 carbon atoms and 2-alkanol having 7 to 9 carbon atoms, and preferably 1,2-alkanediol having 8 to 10 carbon atoms and 2-alkanol having 7 to 9 carbon atoms have a linear carbon chain. The 1,2-alkanediol having 8 to 10 carbon atoms is preferably 1,2-octanediol or 1,2-decanediol. As the 2-alkanol having 7 to 9 carbon atoms, 2-heptanol and 2-octanol are preferable.

The aromatic compound in the compound of the present embodiment is at least one selected from the group consisting of chlorphenesin, 3-phenoxy-1-propanol, 1-(3-methoxyphenoxy)-2-propanol, and orthophenylphenol, preferably at least one selected from the group consisting of chlorphenesin, 3-phenoxy-1-propanol, and orthophenylphenol, and more preferably at least one selected from the group consisting of chlorphenesin and 3-phenoxy-1-propanol.

When the compound of the present embodiment is contained in the measurement sample, even when yeast-like fungi are contained in the urine specimen, it is possible to clearly discriminate between yeast-like fungi and red blood cells. That is, when the measurement sample does not contain the compound of the present embodiment, if yeast-like fungi are present in the urine sample, scattered light information and fluorescence information obtained from yeast-like fungi may overlap with red blood cells, and it may be difficult to discriminate yeast-like fungi and red blood cells. On the other hand, in the analysis method of the present embodiment, since the compound of the present embodiment is contained in the measurement sample, it is possible to damage the cell membrane of yeast-like fungi without hemolysis of red blood cells. As a result, the fluorescent dye is easily permeated into the cell of the yeast-like fungus, and a difference in stainability of the fluorescent dye can be generated between the yeast-like fungus and the red blood cell. As a result, a difference occurs between the fluorescence information detected from red blood cells, and the discrimination accuracy of red blood cells can be improved.

As described above, the first reagent also serves as a diluent for the urine sample, and in the measurement sample, the compound of the present embodiment is present in a mixed state with the urine sample and the second reagent. Accordingly, the compound of the present embodiment may be adjusted such that the concentration (hereinafter, also simply referred to as [final concentration]) in the measurement sample obtained by mixing the first reagent with the urine sample and the second reagent is within a predetermined range, taking into consideration the mixing ratio of the first reagent and the urine sample and the second reagent.

The final concentration of the compound of the present embodiment may be adjusted independently to a range in which red blood cells are substantially not hemolysed and cell membranes of cells such as yeast-like fungi can be appropriately damaged. In addition, the upper limit value of the final concentration of the compound of the present embodiment may be independently set as the saturation concentration of each compound.

The final concentration of the compound of the present embodiment is preferably 0.1 g/L or more. Further, the final concentration is preferably 12 g/L or less.

The final concentration of each aliphatic compound in the compounds of the present embodiment may be, independently, 0.1 to 12 g/L.

The final concentration of 1,2-octanediol is preferably 0.7 g/L or more, more preferably 1 g/L or more, and most preferably 1.2 g/L or more. Further, the final concentration of 1,2-octanediol is preferably 7.3 g/L or less, more preferably 5.8 g/L or less, and most preferably 5 g/L or less.

The final concentration of 1,2-decanediol is preferably 0.1 g/L or more, more preferably 0.3 g/L or more, and most preferably 0.5 g/L or more. Further, the final concentration of 1,2-decanediol is preferably 2 g/L or less, more preferably 1.5 g/L or less, and most preferably 1 g/L or less.

The final concentration of 2-alkanol having 7 to 9 carbon atoms, particularly 2-heptanol or 2-octanol, is preferably 1 g/L or more, more preferably 1.5 g/L or more, and most preferably 2 g/L or more. Further, the final concentration of 2-alkanol having 7 to 9 carbon atoms is preferably 12 g/L or less, more preferably 10 g/L or less, and most preferably 8 g/L or less.

The final concentration of each aromatic compound in the compound of the present embodiment may be 0.8 to 9 g/L each independently.

The final concentration of chlorphenesin is preferably 0.8 g/L or more, more preferably 1 g/L or more, and most preferably 1.2 g/L or more. Further, the final concentration of chlorphenesin is preferably 4 g/L or less, more preferably 3.5 g/L or less, and most preferably 3.2 g/L or less.

The final concentration of orthophenylphenol is preferably 1 g/L or more, more preferably 1.5 g/L or more, and most preferably 2 g/L or more. Further, the final concentration of orthophenylphenol is preferably 6 g/L or less, more preferably 5 g/L or less, and most preferably 3 g/L or less.

The final concentration of 3-phenoxy-1-propanol is preferably 1 g/L or more, more preferably 1.5 g/L or more, and most preferably 2 g/L or more. Further, the final concentration of 3-phenoxy-1-propanol is preferably 8 g/L or less, more preferably 7 g/L or less, and most preferably 6 g/L or less.

The final concentration of 1-(3-methoxyphenoxy)-2-propanol is preferably 4.5 g/L or more, more preferably 5.5 g/L or more, and most preferably 6 g/L or more. Further, the final concentration of 1-(3-methoxyphenoxy)-2-propanol is preferably 9 g/L or less, more preferably 8 g/L or less, and most preferably 7 g/L or less.

The concentration of the compound of the present embodiment in the first reagent (hereinafter, also simply referred to as "concentration in reagent") can be adjusted to be the above-described final concentration in consideration of the mixing ratio with the urine sample and the second reagent. The lower limit of the concentration in the reagent can be appropriately adjusted within a range in which the effect of the present invention occurs. The upper limit of concentration in the reagent may be a concentration at which each compound is saturated.

The concentration of the compound of the present embodiment in the reagent is preferably 0.1 g/L or more. Further, the concentration in the reagent is preferably 15 g/L or less.

The concentration of each aliphatic compound in the compound of the present embodiment in the reagent may be independently 0.1 to 15 g/L.

The concentration of 1,2-octanediol in the reagent is preferably 0.5 g/L or more, more preferably 1 g/L or more, and most preferably 1.5 g/L or more. Also, the concentration of 1,2-octanediol in the reagent is preferably 10 g/L or less, more preferably 8 g/L or less, and most preferably 7 g/L or less.

The concentration of 1,2-decanediol in the reagent is preferably 0.1 g/L or more, more preferably 0.5 g/L or more, and most preferably 0.7 g/L or more. Also, the concentration of 1,2-decanediol in the reagent is preferably 2 g/L or less, more preferably 1.5 g/L or less, and most preferably 1.2 g/L or less.

The concentration of 2-alkanol having 7 to 9 carbon atoms, particularly 2-heptanol or 2-octanol, in the reagent is preferably 1 g/L or more, more preferably 2 g/L or more, and most preferably 3 g/L or more. Further, the concentration of 2-alkanol having 7 to 9 carbon atoms in the reagent is preferably 15 g/L or less, more preferably 13 g/L or less, and most preferably 12 g/L or less.

The concentration of each aromatic compound of the compounds of the present embodiment in the reagent may be independently 0.5 to 15 g/L.

The concentration of chlorphenesin in the reagent is preferably 0.5 g/L or more, more preferably 1 g/L or more, and most preferably 1.5 g/L or more. Also, the concentration of chlorphenesin in the reagent is preferably 10 g/L or less, more preferably 8 g/L or less, and most preferably 6 g/L or less.

The concentration of orthophenylphenol in the reagent is preferably 1 g/L or more, more preferably 2 g/L or more, and most preferably 2.5 g/L or more. Also, the concentration of orthophenylphenol in the reagent is preferably 8 g/L or less, more preferably 6 g/L or less, and most preferably 4 g/L or less.

The concentration of 3-phenoxy-1-propanol in the reagent is preferably 1 g/L or more, more preferably 2 g/L or more, and most preferably 3 g/L or more. The concentration of 3-phenoxy-1-propanol in the reagent is preferably 15 g/L or less, more preferably 10 g/L or less, and most preferably 8 g/L or less.

The concentration of 1-(3-methoxyphenoxy)-2-propanol in the reagent is preferably 4 g/L or more, more preferably 6 g/L or more, and most preferably 8 g/L or more. Also, the concentration of 1-(3-methoxyphenoxy)-2-propanol in the reagent is preferably 15 g/L or less, more preferably 12 g/L or less, and most preferably 10 g/L or less.

The first reagent may contain an aliphatic compound and/or an aromatic compound other than the compound of the present embodiment. For example, the first reagent may contain an aromatic compound (hereinafter, also referred to as [another aromatic compound]) that can damage the cell membrane of yeast-like fungi. Examples of the other aromatic compound include aromatic alcohols such as benzyl alcohol, β-phenotyl alcohol, phenol, 1-phenoxy-2-propanol, and 2-phenoxyethanol; phenyl acetate; and benzothiazole compounds such as 2-aminobenzothiazole and benzothiazole. The other aromatic compound may be used singly or in combination of two or more.

When the first reagent contains another aromatic compound, the total final concentration of the other aromatic compound and the compound of the present embodiment is preferably 1 g/L or more. Further, the total of the final concentrations is preferably 12 g/L or less. Further, the total concentration in the reagent is preferably 1 g/L or more. Further, the total concentration in the reagent is preferably 15 g/L or less. In this case, the final concentration of the compound of the present embodiment is preferably 0.1 times or more, more preferably 0.2 times or more, and most preferably 0.3 times or more, relative to the final concentration of the other aromatic compound. Further, the final concentration of the compound of the present embodiment is preferably 100 times or less, more preferably 10 times or less, and most preferably 5 times or less, relative to the final concentration of another aromatic compound. Even when the first reagent contains another aromatic compound, the final concentration of the compound of the present embodiment and the concentration in the reagent may be within the ranges described above as preferred ranges, respectively.

The first reagent may further comprise a component other than the component described above. Such components include, for example, solvents, surfactants, buffers, osmotic pressure compensators, and chelating agents.

Solvents include, for example, water, water-soluble organic solvents, and mixtures thereof. Examples of the water-soluble organic solvent include lower alcohols having 1 to 3 carbon atoms, ethylene glycol, and dimethylsulfoxide (DMSO). Preferably, the solvent is water.

Surfactants may be formulated to damage the cell membrane of contaminants and/or to enhance the solubility of the compounds of this embodiment. The type of surfactant is not particularly limited, and may be appropriately selected from a cationic surfactant, a nonionic surfactant, an anionic surfactant, and an amphoteric surfactant. One surfactant may be used alone, or two or more surfactants may be used in combination.

For instance, the cationic surfactants include quaternary ammonium salt-type surfactants and pyridinium salt-type surfactants. Specifically, the examples of the cationic surfactants include the following compounds:
Triethylmethylammonium chloride
Didecyldimethylammonium chloride
Didecyldimethylammonium bromide
Dodecyldimethylammonium bromide
Didodecyldimethylammonium bromide
Didodecyldimethylammonium chloride
Ethylhexadecyldimethylammonium bromide
Ethyltrimethylammonium iodide
Dodecyltrimethylammonium bromide
Decyltrimethylammonium bromide
Dodecyltrimethylammonium chloride
Octyltrimethylammonium bromide
Octyltrimethylammonium chloride
Tetradecyltrimethylammonium chloride
Trimethylheptadecylammonium bromide
Hexadecyltrimethylammonium bromide
Hexadecyltrimethylammonium chloride
Hexadecyltrimethylammonium hydroxide
Hexyltrimethylammonium bromide
Trimethylnonylammonium bromide
Trimethylstearylammonium bromide
Trimethylstearylammonium chloride
Dodecylpyridinium chloride
Tetradecyltrimethylammonium bromide

Examples of the nonionic surfactant include a polyoxyethylene-based nonionic surfactant. Specific examples thereof include polyoxyethylene alkyl ether, polyoxyethylene sterol, polyoxyethylene castor oil, polyoxyethylene sorbit fatty acid ester, polyoxyethylene alkyl amine, and polyoxyethylene polyoxypropylene alkyl ether.

Anionic surfactants include carboxylate-type surfactants, sulfonate-type surfactants, and sulfate ester-type surfactants. Specifically, the examples of the anionic surfactants include the following compounds:
Carboxylate surfactant
Sulfonate surfactant
Sulfate ester surfactant
Sodium laurate
Sodium stearate
Sodium oleate
Linear decylbenzene sulfonate sodium salt
Linear undecylbenzene sulfonate sodium salt
Linear dodecylbenzene sulfonate sodium salt
Linear tridecylbenzene sulfonate sodium salt
Linear tetradecylbenzene sulfonate sodium salt
1-Tetradecenesulfonic acid sodium salt
Hexadecenesulfonic acid sodium salt
3-Hydroxyhexadecyl-1-sulfonic acid sodium salt
1-Octadecenesulfonic acid sodium salt
3-Hydroxy-1-octadecylsulfonic acid sodium salt
Sodium decyl sulfate
Sodium undecyl sulfate
Sodium dodecyl sulfate
Sodium tridecyl sulfate
Sodium tetradecyl sulfate
Sodium dodecyl ether sulfate
2-Sulfo-tetradecanoic acid-1-methyl ester sodium salt
2-Sulfo-hexadecanoic acid-1-methyl ester sodium salt

Amphoteric surfactants include amino acid-type amphoteric surfactants and betaine-type amphoteric surfactants. Specific examples thereof include 3-(dodecylamino)propanoic acid, 3-(tetradeca-1-ylamino)propanoic acid, and the like; dodecyldimethylaminoacetic acid betaine, stearyl dimethylaminoacetic acid betaine, and the like.

The surfactant may be contained in the first reagent such that the final concentration in the prepared measurement sample is, for example, 2 to 30 mg/L, preferably 4 to 22 mg/L.

A buffer may be formulated to keep the pH of the first reagent constant. The pH of the first reagent is preferably 6.0 to 9.0, and more preferably 7.5 to 9.0. By keeping the pH of the first reagent constant, hemolysis of red blood cells due to a pH change tends to be suppressed. The buffering agent is not particularly limited as long as it has a buffering action within the above-described pH range, and examples thereof include good buffering agents such as Tris,MES,Bis-Tris,ADA,PIPES,ACES,MOPS,MOPSO,BES,TES,HEPES,DIPSO,TAPSO,POPSO, HEPPSO,EPPS,Tricine,Bicine, and TAPS.

An osmotic pressure compensating agent may be formulated to inhibit hemolysis of red blood cells and obtain stable fluorescence information. Since the osmotic pressure of urine is distributed over a wide range of 50 to 1300 mOsm/kg, the osmotic pressure of the first reagent is preferably 100 to 1200 mOsm/kg, and more preferably 200 to 800 mOsm/kg. When the osmotic pressure is within the above range, hemolysis of red blood cells tends to be suppressed and stable fluorescence information can be obtained.

Examples of the osmotic pressure compensating agent include inorganic salts, organic salts such as propionate, and saccharides. Inorganic salts include sodium chloride, potassium chloride, sodium bromide, and the like. Among the organic salts, examples of the propionate include sodium propionate, potassium propionate, and ammonium propionate. Other organic salts include oxalate, acetate, and the like. As the saccharide, sorbitol, glucose, mannitol, and the like are used. One type of osmotic pressure compensator may be used alone, or two or more types may be used in combination.

Chelating agents may be formulated to reduce the effect of amorphous salts (e.g., ammonium phosphate, magnesium phosphate, calcium carbonate, etc.) appearing in urine. Chelating agents include decalcifying agents and demagnesium removing agents. As used herein, the term "decalcifying agent" and "de-magnesium agent" refer to those that combine with calcium ions and magnesium ions, respectively, to form a water-soluble compound. Specific examples of the chelating agent include EDTA-salt, CyDTA,DHEG,DPTA-OH,EDDA,EDDP,GEDTA,HDTA,HIDA,Methyl-EDTA,NTA,NTP,NTPO, and EDDPO. Preference is given to using EDTA-salt, CyDTA,GEDTA. The chelating agent may be used singly or in combination of two or more. The chelating agent may be formulated so that the concentration in the first reagent is, for example, 0.05 to 5 W/W %, preferably 0.1 to 1 W/W %.

The second reagent is a reagent comprising a fluorescent dye that stains nucleic acids or cytoplasm. Even when the first reagent comprises a compound of the present embodiment and the second reagent comprises such a fluorescent dye in a urine specimen, even when yeast-like fungi are contained in the urine specimen, the fluorescent dye permeates the cell interior of the yeast-like fungi after damaging the cell membrane of the yeast-like fungi, and stains nucleic acids or cytoplasm inside the cell. Accordingly, it is possible to cause a difference in staining property of a fluorescent dye between yeast-like fungi and red blood cells, and it is possible to improve discrimination accuracy of red blood cells.

Such a fluorescent dye may stain nucleic acids or cytoplasm, but may stain a cell membrane, a membrane protein of a cell membrane, or the like. Preferably, such fluorescent dye stains nucleic acids. Since red blood cells do not have nucleic acids, whereas yeast-like fungi have nucleic acids, the discrimination accuracy of red blood cells can be further improved by using such a fluorescent dye.

In addition to the fluorescent dye that stains nucleic acids or cytoplasm, the second reagent may include a fluorescent dye that is capable of staining cell membranes, preferably proteins and cell membranes of red blood cells, and the fluorescent dye that stains nucleic acids or cytoplasm described above may stain cell membranes, preferably cell membranes of proteins and red blood cells.

Examples of the fluorescent dye that can be used in the second reagent include a condensed benzene derivative, and among the derivatives, a cyanine-based fluorescent dye is particularly preferable. Examples of the cyanine-based fluorescent dye include 3,3'-diethyloxacarbocyanine iodide (DiOC2(3)), 3,3'-dipropyloxacarbocyanine iodide (DiOC3(3)), 3,3'-dibutyloxacyanine iodide (DiOC4(3)), and 3,3'-dipentylcarbocyanine iodide (DiOC5(3)). Such a fluorescent dye is a fluorescent dye capable of staining the cell membrane of red blood cells in addition to nucleic acids or cytoplasm.

One fluorescent dye may be used alone, or two or more of them may be used in combination. Preferably, the concentration of the fluorescent dye in the second reagent is set so that the fluorescent dye is contained in the prepared measurement sample at a final concentration such that red blood cells can be appropriately stained. The final concentration in the measurement sample is appropriately set according to the type of the fluorescent dye described above. For example, when DiOC3(3) is used as the fluorescent dye, the final concentration in the measurement sample may be, for example, 0.1 to 200 µg/mL, and preferably 1 to 20 µg/mL.

The second reagent may include a component other than the fluorescent dye described above. Such components include a solvent. The solvent is not particularly limited as long as it is an aqueous solvent capable of dissolving the fluorescent dye, and examples thereof include water, a water-soluble organic solvent, and a mixture thereof. Of these, a water-soluble organic solvent is particularly preferred. Examples of the water-soluble organic solvent include lower alcohols having 1 to 3 carbon atoms, ethylene glycol, and DMSO.

In the measurement sample preparation step S1, the measurement sample is prepared by mixing the urine sample, the first reagent, and the second reagent described above. The order in which the urine sample, the first reagent, and the second reagent are mixed is not particularly limited, and may be mixed at the same time. Preferably, the urine sample and the second reagent are mixed first and the first reagent is further mixed therein. Alternatively, the first reagent and the second reagent may be mixed first, and the urine sample may be further mixed therein.

The mixing ratio of the urine sample, the first reagent, and the second reagent is not particularly limited, and may be appropriately determined according to the concentration of components contained in each reagent. For example, the mixing ratio of the urine sample to the first reagent can be determined from the range of 1:0.5 to 1:10 by volume ratio. Also, the mixing ratio of the urine sample to the second reagent can be determined from the range of 1:0.01 to 1:1 by volume ratio. Note that the amount of the urine sample may be appropriately determined according to the first reagent and the second reagent. The amount of the urine sample is preferably 1000 µL or less from the viewpoint of preventing the measurement time from becoming too long. The amount of the urine sample is about 10 to 1000 µL, which is sufficient for measurement.

In the measurement sample preparation step S1, the urine sample, the first reagent, and the second reagent may be mixed while being heated. The mixing temperature may be, for example, 10 to 60° C., preferably 35 to 45° C. Each reagent may be previously warmed to be at these temperatures. Further, the stirring time after mixing the urine sample with the first reagent and/or the second reagent may be, for example, 1 second to 5 minutes, and preferably 5 to 60 seconds.

### (measurement sample acquisition step)

Next, in the analysis method according to the first embodiment, the measurement apparatus that performs step S3, which will be described later, which acquires particle information from the measurement sample, acquires the measurement sample prepared in step S1 (step S2). Accordingly, in the measurement sample obtaining step S2, a measurement sample comprising: the urine sample; at least one compound selected from the group consisting of aliphatic compound and aromatic compound; and a fluorescent dye that stains nucleic acids or cytoplasm is obtained.

Note that step S1 may be performed by the analyzer, or may be performed by the above-described measurement apparatus in which each sample and reagent are set. If step S1 is performed by the analyst or another apparatus connected to the measurement apparatus described above is performed, in the measurement sample acquisition step S2, the measurement apparatus acquires the measurement sample prepared in step S1 from the outside. When the measurement apparatus performs step S1, in the measurement sample acquisition step S2, the measurement apparatus acquires the measurement sample from the storage unit in which the measurement sample prepared in step S1 is stored, by the analysis unit for analyzing the measurement sample.

### (Particle information acquisition step)

Next, in the analysis method according to the first embodiment, the measurement device irradiates the measurement sample with light and acquires fluorescence information of particles contained in the measurement sample (step S3).

The measurement apparatus is not particularly limited as long as it is an apparatus capable of obtaining the measurement sample, irradiating the measurement sample with light, obtaining fluorescence information of particles contained in the measurement sample, and detecting red blood cells based on the fluorescence information. Such devices include flow cytometers. In measurement by a flow cytometer, optical information can be obtained as a signal emitted from a urine formed component stained with a fluorescent dye by irradiating the formed component with light when the urine formed component passes through a flow cell. The obtained optical information includes at least fluorescence information.

The fluorescence information is not particularly limited as long as it is information obtained by irradiating the stained urine formed component with excitation light having an appropriate wavelength and measuring the excited fluorescence, and examples thereof include intensity and waveform information of fluorescence. More specifically, the fluorescence information includes fluorescence intensity, fluorescence pulse width, fluorescence integration value, and the like. In this embodiment, since the compound of the present embodiment increases the fluorescence intensity of contaminants such as yeast-like fungi, it is preferable that the fluorescence information includes the fluorescence intensity. When the fluorescence information includes fluorescence intensity, fluorescence information from red blood cells can be accurately acquired.

Preferably, the analysis method according to the present embodiment further includes the step of obtaining scattered light information of particles contained in the measurement sample irradiated with light simultaneously with the step 3 of obtaining fluorescence information of particles or after the step 3. When the flow cytometer is used as the measurement apparatus for obtaining fluorescence information, in step 3 of obtaining fluorescence information of particles, scattered light information of particles can be obtained at the same time as the fluorescence information is obtained.

The scattered light information may be, for example, information of scattered light that can be measured by a flow cytometer, and examples thereof include intensity of scattered light such as forward scattered light (for example, around a light receiving angle of 0 to 20 degrees) and side scattered light (around a light receiving angle of 90 degrees), waveform information, and the like. More specifically, the scattered light information includes scattered light intensity, scattered light pulse width, scattered light integration value, and the like. Side scattered light reflects information inside particles, such as nuclei and granules of cells, and forward scattered light tends to reflect information on particle size. In the present embodiment, in order to accurately discriminate red blood cells from cells such as yeast-like fungi, the scattered light information preferably includes forward scattered light information, and more preferably includes forward scattered light intensity.

**In** the present embodiment, the light to be irradiated onto the measurement sample, that is, the light source provided in the measurement apparatus is not particularly limited, and a light source having a wavelength suitable for excitation of the fluorescent dye contained in the measurement sample can be appropriately selected. For example, a red semiconductor laser, a blue semiconductor laser, an argon laser, a He-Ne laser, a mercury arc lamp, or the like may be used.

### (red blood cell detection step)

Next, in the analysis method according to the first embodiment, the measurement apparatus detects red blood cells based on the fluorescence information acquired in step S3 (step S4). **In** the present specification, "detection" includes not only finding the presence of a formed element in urine in a measurement sample but also classifying and counting a formed element in urine.

In the red blood cell detection step S4, the red blood cell may be detected based on the fluorescence information acquired in step S3, and for example, particles having predetermined fluorescence information may be detected as red blood cells. More specifically, particles having a fluorescence intensity in a predetermined range can be detected as red blood cells. In the analysis method according to the present embodiment, since a difference in staining property of a fluorescent dye is generated between yeast-like fungi and red blood cells as described above, red blood cells can be accurately detected by setting an appropriate fluorescence intensity also in a urine specimen containing yeast-like fungi. The fluorescence information for detection as red blood cells can be determined based on fluorescence information obtained from particles in urine that have been confirmed to be red blood cells in advance by microscopy or the like.

From the viewpoint of enhancing detection accuracy of red blood cells, in the red blood cell detection step S4, it is preferable to detect red blood cells based on the fluorescence information and the scattered light information. By detecting red blood cells based on the scattered light information as described above, red blood cells can be detected based on the size of particles and internal information.

In the red blood cell detection step S4, a scattergram having scattered light information and fluorescence information as two axes is generated, and the obtained scattergram may be analyzed using appropriate analysis software. For example, when a scattergram is drawn with the X axis as the fluorescence intensity and the Y axis as the forward scattered light intensity, a respective population (cluster) appears on the scattergram depending on the particle size and staining property of each urine concrete component. In the analysis method according to the present embodiment, at least yeast-like fungi and red blood cells can be detected as two populations each appearing in a different region. Also, the analysis software can provide a window surrounding each population on the scattergram and count the number of particles in each window. The region where red blood cells and yeast-like fungi appear can be determined based on the forward scattered light intensity and fluorescence intensity obtained from particles in urine that have been previously confirmed to be red blood cells and yeast-like fungi by microscopic examination or the like.

### (The second embodiment)

A method for analyzing a urine sample according to the second embodiment includes the steps of obtaining a measurement sample comprising: the urine sample; at least one compound selected from the group consisting of aliphatic compound and aromatic compound; and a fluorescent dye that stains nucleic acids or cytoplasm; irradiating the measurement sample with light and obtaining fluorescence information of particles contained in the measurement sample; and discriminating between red blood cells and yeast-like fungi based on the fluorescence information.

FIG. 2 is a flowchart illustrating an example of a method for analyzing a urine sample according to the second embodiment. As shown in FIG. 2, in the urine sample analysis method according to the second embodiment, the measurement sample is prepared (step S1), the measurement apparatus obtains the measurement sample (step S2), obtains particle information from the measurement sample (step S3), and discriminates between red blood cells and yeast-like fungi based on the obtained particle information (step S5).

Steps S1 to S3 in the analysis method according to the second embodiment can be performed in the same manner as steps S1 to S3 in the analysis method according to the first embodiment. Hereinafter, a step S5 of discriminating between red blood cells and yeast-like fungi will be described, and a description that overlaps with the analysis method according to the first embodiment will be omitted.

### (red blood cell discrimination step)

In the analysis method according to the second embodiment, red blood cells and yeast-like fungi are discriminated from each other based on the fluorescence information acquired in step S3 (step S5). By discriminating between red blood cells and yeast-like fungi is meant detecting at least one of red blood cells and yeast-like fungi while discriminating between red blood cells and yeast-like fungi.

In the red blood cell discrimination step S5, it is sufficient to discriminate between red blood cells and yeast-like fungi based on the fluorescence information acquired in step S3, and for example, it is sufficient to make particles having predetermined fluorescence information red blood cells and discriminate particles having predetermined fluorescence information from yeast-like fungi. More specifically, particles having a fluorescence intensity in a predetermined range can be determined as red blood cells, and particles having a fluorescence intensity in a predetermined range higher than the predetermined range can be determined as yeast-like fungi.

From the viewpoint of increasing the discrimination accuracy of red blood cells, in the red blood cell discrimination step S5, it is preferable to discriminate between red blood cells and yeast-like fungi based on the fluorescence information and the scattered light information. By discriminating between red blood cells and yeast-like fungi based on the scattered light information as described above, it is possible to discriminate between red blood cells and yeast-like fungi based on the particle size and internal information.

In the red blood cell discrimination step S5, a scattergram having scattered light information and fluorescence information as two axes is generated, and the obtained scattergram may be analyzed using appropriate analysis software. For example, if a scattergram is drawn with the X-axis as the fluorescence intensity and the Y-axis as the forward scattered light intensity, yeast-like fungi and red blood cells can be discriminated as two populations each appearing in a different region. After discriminating between red blood cells and yeast-like fungi in this manner, the analysis software may provide a window around each population on the scattergram and count the number of particles in each window.

### [reagent for urine sample analysis]

The reagent for urine sample analysis according to the present embodiment is a reagent for detecting at least red blood cells as a formed component in a urine sample, and includes the compound of the present embodiment described above. Preferred embodiments of the aliphatic compound and the aromatic compound in the compound of the present embodiment are as described above.

The reagent for urine sample analysis according to the present embodiment may contain the compound so that the final concentration of the compound of the present embodiment is within the above range when mixed with a urine sample or the like. The concentration of the compound of the present embodiment in the reagent for urine sample analysis according to the present embodiment may be within the range described above as the concentration of the compound in the first reagent.

The reagent for urine sample analysis according to the present embodiment is preferably a dilution reagent for urine sample, and more preferably the reagent described above as the first reagent. Therefore, the reagent for urine sample analysis according to the present embodiment may contain the same component as the first reagent described above at the same concentration.

### [reagent kit for urine sample analysis]

The reagent kit for urine sample analysis according to the present embodiment is a kit used for detecting at least red blood cells as a formed component in a urine sample, and includes, in addition to the reagent for urine sample analysis according to the present embodiment, a reagent comprising a fluorescent dye that stains nucleic acids or cytoplasm. A reagent comprising a fluorescent dye may comprise components similar to the second reagent described above in a similar concentration. The reagent kit for urine sample analysis according to the present embodiment may be a kit comprising the first reagent and the second reagent described above.

In the reagent kit for urine sample analysis according to the present embodiment, it is preferable that the first reagent and the second reagent are contained in containers separate from each other, and a reagent kit of a two-reagent type including the first reagent and the second reagent is provided.

### [Example]

Hereinafter, the present invention will be described in more detail with reference to Examples. The present invention is not limited in any way by the following examples.

### [Example 1]

In Example 1, the effect of staining of yeast-like fungi by the compound added to the first reagent was examined.

### (1) First reagent (dilution reagent)

Each of the following substances was dissolved in purified water to prepare a buffer solution.

**[Table 1]**

| SUBSTANCE NAME | CONCENTRATION (g/L ) |
|---|---|
| TRIS BUFFER | 6.1 |
| EDTA-2K | 12.13 |
| SODIUM CHLORIDE | 10 |
| HYDROCHLORIC ACID | ADJUST pH TO ABOUT 8 |

In this example, the compound shown in the following table was added to the buffer solution so as to have a concentration shown in the following table, and a first reagent was prepared. When the compound shown in the following table was not dissolved, the compound was used after filtration. The osmolality of the reagents in the table was about 410-520 mOsm/kg. Furthermore, a first reagent having the same composition as Diluting Liquid of EXAMPLE-1 of U.S. Patent Publication No. 2006/0073601 was prepared. The first reagent is represented by [2-phenoxyethanol(*)] in the following table. In the following table, the compound of the present embodiment is shown in bold.

**[Table 2]**

| TYPE | | ADDED COMPOUND | CONCENTRATION [g/L] | |
|---|---|---|---|---|
| | | | CONCENTRAT ION IN FIRST REAGENT | FINAL CONCENTRAT ION |
| NOT ADDED | | | 0 | 0 |
| AROMATIC COMPOUND | | **3-PHENOXY-1-PROPANOL** | 7 | 5.1 |
| | | **1-(3-METHOXYPHENOX Y)-2-PROPANOL** | 9 | 6.5 |
| | | **ORTHOPHENYLPH ENOL** | 3 | 2.2 |
| | | **CHLORPHENESIN** | 3 | 2.2 |
| | | 2-PHENOXYETHANOL | 7.5 | 5.4 |
| | | 2-PHENOXYETHANOL (*) | 7.5 | 5.4 |
| | | PHENYL DIGLYCOL | 10 | 7.3 |
| | | METHYLPARABEN | 1.9 | 1.4 |
| | MONOVAL ENT | **2-HEPTANOL** | 10 | 7.3 |
| | | **2-OCTANOL** | 10 | 7.3 |
| | | 2-DECANOL | 10 | 7.3 |
| | BIVALENT | **1,2-OCTANEDIOL** | 6 | 4.4 |
| ALIPHATI C COMPOU ND | | **1,2-DECANEDIOL** | 1 | 0.7 |
| | | 1,2-HEXANEDIOL | 10 | 7.3 |
| | | 1,2-DODECANEDIOL | 0.5 | 0.4 |
| | | 1,8-OCTANEDIOL | 10 | 7.3 |
| | | 1,2,6-HEXANETRIOL | 10 | 7.3 |

### (2) A second reagent (staining reagent)

UF-fluorocell SF for UF-5000 (Sysmex Corporation) was used. Such reagents include polymethine-based dyes and stain lipid bilayer membranes and porous protein aggregates of cells, and the like.

### (3) Urine sample

A purified yeast fungus (*C*. *glabrata*) was added to negative urine to a concentration of about 1800 cells/µL. In order to confirm the effect on red blood cells, human blood was added to negative urine so that the concentration of red blood cells was about 400 cells/µL. As the negative urine, a commercially available Normal Human Urine (Golden West Diagnostics,LLC) was filtered through a 0.22-µm membrane filter, and a urine specimen having a negative result in a sediment assay was used.

### (4) Measurement and result

A measurement sample was prepared by mixing a first reagent and a second reagent with a urine sample, and measurement was performed using an SF channel of a flow cytometer UF-5000 (Sysmex Corporation), and a scattergram was prepared in which a forward scattered light intensity was taken on the vertical axis and a fluorescence intensity was taken on the horizontal axis. In measurement using the SF channel, red blood cells, crystals, a glass cylinder, a cylinder containing contents, and the like are measured. To calculate the average value of the fluorescence intensity of yeast fungi, an arbitrary region Y for each measurement was set, and the average value of the fluorescence intensity of a population of dots appearing in the region was calculated. Note that the value of the fluorescence intensity is a relative value when the minimum value of the horizontal axis in the scattergram is 0 and the maximum value is 255.

Flow cytometer measurements were performed according to the UF-5000 manual except that the prepared first reagent was used instead of UF-CELLPACK SF. Specifically, after mixing 362.5 µL of the first reagent, 125 µL of the urine specimen, and 12.5 µL of the second reagent, the measurement sample was prepared by stirring at about 39° C. for 9 seconds, and then flowed into the flow cell of the detection section. Information on scattered light and fluorescence emitted from particles in the measurement sample that received the laser beam was acquired by the detection unit. A scattergram was generated based on the obtained optical information.

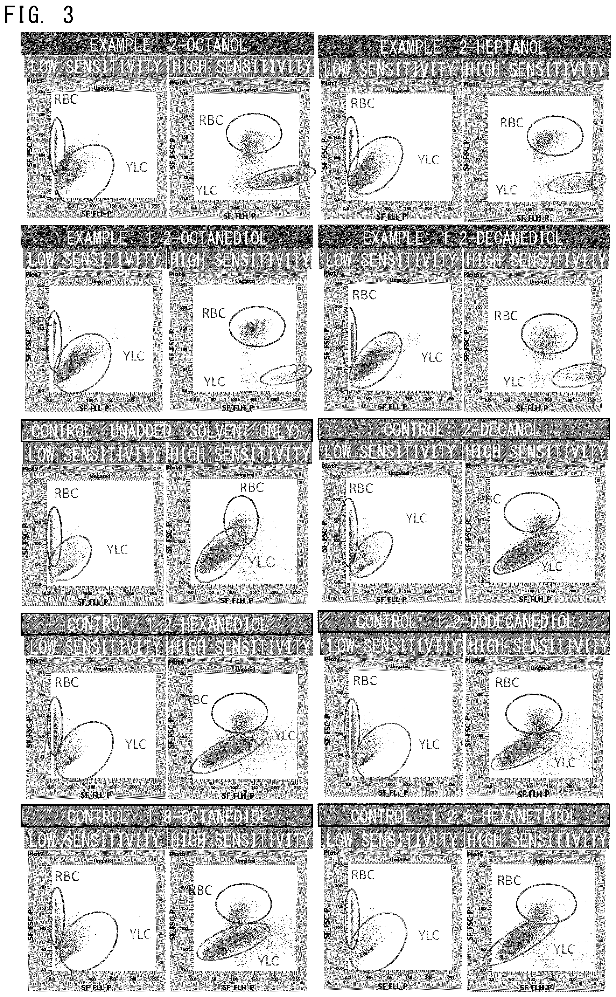

The generated scattergram is shown in FIGS. 3 to 4. In the scattergram shown below, the horizontal axis represents fluorescence intensity, and the higher the value is to the right, the higher the fluorescence intensity. The vertical axis is the forward scattered light intensity and means that the higher the value is, the higher the forward scattered light intensity is. Also, RBC means red blood cells, and YLC means yeast-like fungi. In addition, a scattergram labeled "high sensitivity" is obtained by expanding a region having a low fluorescence intensity in a scattergram labeled "low sensitivity". 3 and 4, when the compound of the present embodiment was used, the yeast-like fungal cluster was located in a region having a higher fluorescence intensity than the control, and the position of the red blood cell cluster was not significantly changed. It has been shown that by using the compound of the present embodiment, the discrimination performance between red blood cells and yeast-like fungi is improved, and red blood cells can be detected with higher accuracy.

Further, the average value of the fluorescence signal intensities of the respective compounds added to the first reagent is shown in the following table. As shown in the following table, when 1,2-alkanediol having 8 to 10 carbon atoms, 2-alkanol having 7 to 9 carbon atoms, and chlorphenesin, 3-phenoxy-1-propanol, 1-(3-methoxyphenoxy)-2-propanol, and orthophenylphenol are used, the average value of the fluorescence intensity is significantly larger and the stainability of yeast-like fungi is improved than when other compounds are used. In the following table, the larger the average value of the fluorescence intensity is, the higher the staining property of yeast-like fungi is, and the higher the discrimination property with respect to red blood cells is. In addition, in the following table, results corresponding to examples containing the compound of the present embodiment are shown in bold. As shown in Table 3, it was found that when the compound of the present embodiment was used, the average value of the fluorescence intensity of the yeast-like fungal cluster was higher than that of the control.

**[Table 3]**

| TYPE | | ADDED COMPOUND | AVERAGE VALUE OF FLUORESCENT SIGNAL INTENSITY [-] |
|---|---|---|---|
| NOT ADDED | | | 83.1 |
| AROMATIC COMPOUND | | 3-PHENOXY-1-PROPANOL | **253.6** |
| | | 1-(3-METHOXYPHENOXY)-2-PROPANOL | **246.8** |
| | | ORTHOPHENYLPHENOL | **250.1** |
| | | CHLORPHENESIN | **251.2** |
| | | 2-PHENOXYETHANOL | 196.9 |
| | | 2-PHENOXYETHANOL (*) | 164.8 |
| | | PHENYL DIGLYCOL | 165.0 |
| | | METHYLPARABEN | 220.5 |
| | MONOVALENT | 2-HEPTANOL | **250.1** |
| | | 2-OCTANOL | **240.6** |
| | | 2-DECANOL | 106.0 |
| | BIVALENT | 1,2-OCTANEDIOL | **253.4** |
| ALIPHATIC COMPOUND | | 1,2-DECANEDIOL | **252.7** |
| | | 1,2-HEXANEDIOL | 107.7 |
| | | 1,2-DODECANEDIOL | 107.6 |
| | | 1,8-OCTANEDIOL | 120.5 |
| | | 1,2,6-HEXANETRIOL | 80.5 |

### [Example 2]

In Example 2, the stainability of yeast-like fungi was examined when two compounds were added to the first reagent.

A urine sample was measured in the same manner as in Example 1, except that the first reagent was prepared by adding the compound of the following table to the buffer solution described in Example 1. The first reagent represented by [2-phenoxyethanol(*)] had the same composition as Diluting Liquid of EXAMPLE-1 of U.S. Publication No. 2006/0073601. When the compound shown in the following table was not dissolved, the compound was used after filtration. The osmolality of the reagents in the table was about 420-500 mOsm/kg. In the following table, the compound of the present embodiment is shown in bold.

**[Table 4]**

| TYPE | ADDED COMPOUND | CONCENTRATION [g/L] | |
|---|---|---|---|
| | | CONCENTRATION IN FIRST REAGENT | FINAL CONCENTRATION |
| CONTROL | 2-PHENOXYETHANOL (*) | 7.5 | 5.4 |
| MIXTURE OF AROMATIC COMPOUNDS | 2-PHENOXYETHANOL | 6 | 4.4 |
| | **CHLORPHENESIN** | 2 | 1.5 |
| | 2-PHENOXYETHANOL | 6 | 4.4 |
| | **3-PHENOXY-1-PROPANOL** | 4 | 2.9 |
| MIXTURE OF ALIPHATIC COMPOUND AND AROMATIC COMPOUND | 2-PHENOXYETHANOL | 6 | 4.4 |
| | **1,2-OCTANEDIOL (FINAL CONCENTRATION 2.2 g/L)** | | |
| | | 3 | 2.2 |
| | 2-PHENOXYETHANOL | 6 | 4.4 |
| | **1,2-OCTANEDIOL (FINAL CONCENTRATION 1.5 g/L)** | | |
| | | 2 | 1.5 |
| | 2-PHENOXYETHANOL | 6 | 4.4 |
| | **2-HEPTANOL** | 4 | 2.9 |

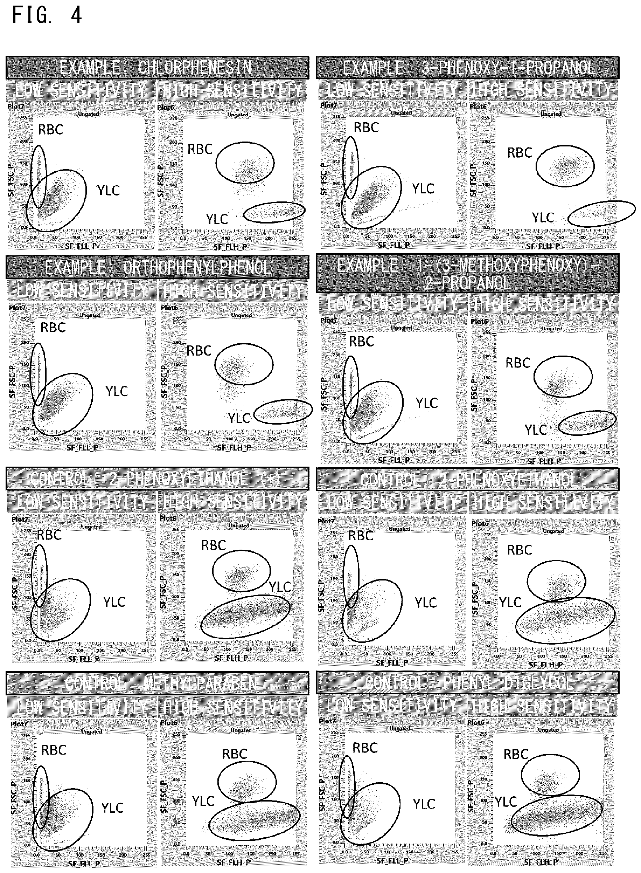

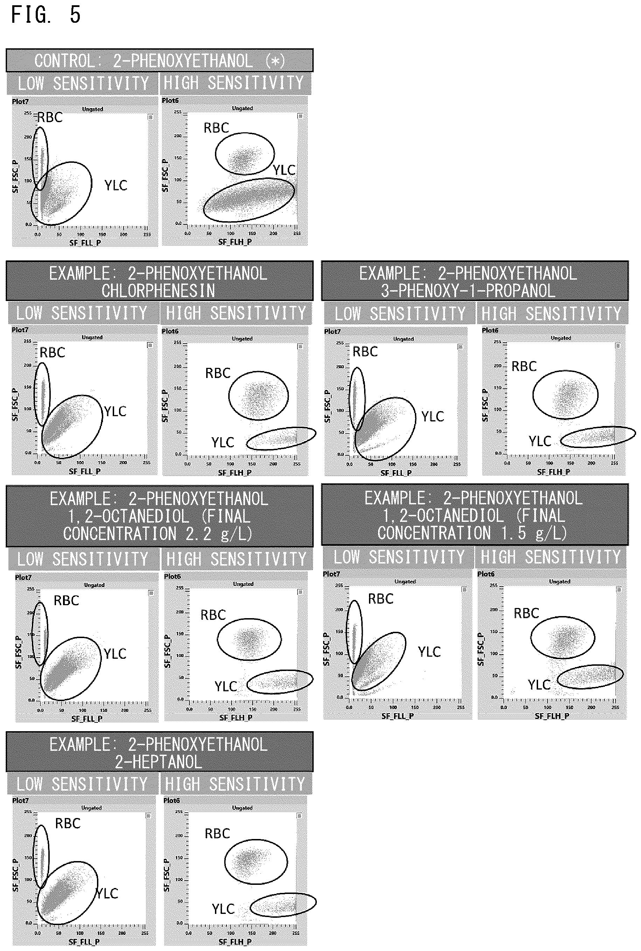

The generated scattergram is shown in FIG. 5. The scattergram of the control reagent [2-phenoxyethanol(*)] is the same as the scattergram of [2-phenoxyethanol(*)] shown in FIG. 4. FIG. 5 shows that by using the compound of the present embodiment, the discrimination performance between red blood cells and yeast-like fungi is improved, and that red blood cells can be detected with higher accuracy. Further, the average value of the fluorescence signal intensities of the respective compounds added to the first reagent is shown in the following table. As shown in the following table, when chlorphenesin, 3-phenoxy-1-propanol, 1,2-octanediol, or 2-heptanol was mixed with 2-phenoxyethanol, the average value of the fluorescence intensity was significantly larger than that when 2-phenoxyethanol was used alone, and the stainability of yeast-like fungi was improved. In the following table, results corresponding to examples containing the compound of the present embodiment are shown in bold.

**[Table 5]**

| TYPE | ADDED COMPOUND | AVERAGE VALUE OF FLUORESCENT SIGNAL INTENSITY [-] |
|---|---|---|
| CONTROL | 2-PHENOXYETHANOL (*) | 164.8 |
| MIXTURE OF AROMATIC COMPOUNDS | 2-PHENOXYETHANOL CHLORPHENESIN | **252.8** |
| | 2-PHENOXYETHANOL | **251.5** |
| | 3-PHENOXY-1-PROPANOL | |
| MIXTURE OF ALIPHATIC COMPOUND AND AROMATIC COMPOUND | 2-PHENOXYETHANOL | **251.9** |
| | 1,2-OCTANEDIOL (FINAL CONCENTRATION 2.2 g/L) | |
| | 2-PHENOXYETHANOL | **250.5** |
| | 1,2-OCTANEDIOL (FINAL CONCENTRATION 1.5 g/L) | |
| | 2-PHENOXYETHANOL | **252.2** |
| | 2-HEPTANOL | |

### [Example 3]

In Example 3, the effect of staining of yeast-like fungi by the concentration of compound added to the first reagent was examined.

Measurement of the urine sample was performed in the same manner as in Example 1, except that the first reagent was prepared by adding the compound of the following table to the buffer solution described in Example 1 at the concentration shown in the following table. When chlorphenesin was added at a concentration of 4 g/L, it exceeded the saturation concentration and did not dissolve, so that the filtrate after filtration was used as the first reagent. Therefore, in Table 6, the concentration of chlorphenesin in the reagent of the first reagent is described as [about 4 g/L], and the final concentration of chlorphenesin in the case where the first reagent is used is described as [about 2.9 g/L].

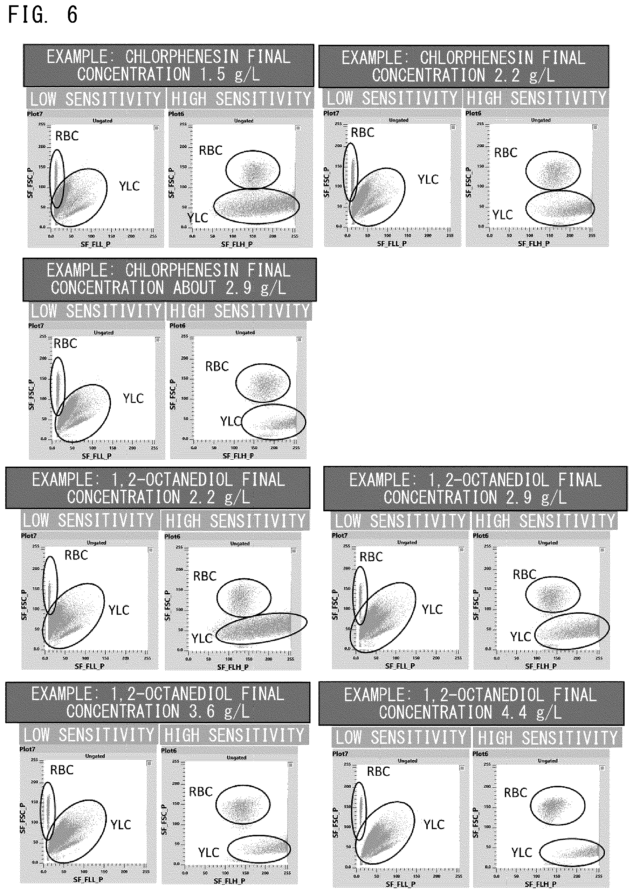

The generated scattergram is shown in FIG. 6 (the concentration designation in FIG. 6 represents the final concentration of each compound). FIG. 6 shows that by using the compound of the present embodiment, the discrimination performance between red blood cells and yeast-like fungi is improved, and that red blood cells can be detected with higher accuracy. Further, the average value of the fluorescence signal intensities of the respective compounds added to the first reagent is shown in the following table. As shown in the following table, as the addition amount of the compound was increased, the average value of the fluorescence intensity was increased, and the stainability of yeast-like fungi was improved. In the following table, results corresponding to examples containing the compound of the present embodiment are shown in bold.

**[Table 6]**

| TYPE | ADDED COMPOUND | CONCENTRATION [g/L] | | AVERAGE VALUE OF FLUORESCE NT SIGNAL INTENSITY [-] |
|---|---|---|---|---|
| | | CONCENTRATI ON IN FIRST REAGENT | FINAL CONCENTRATI ON | |
| AROMATI C COMPOU ND | CHLORPHENES IN | 2 | 1.5 | **230.6** |
| | | 3 | 2.2 | **248.3** |
| | | ABOUT 4 | ABOUT 2.9 | **252.1** |
| ALIPHATI C COMPOU ND | 1,2-OCTANEDIOL | 3 | 2.2 | **222.1** |
| | | 4 | 2.9 | **245.3** |
| | | 5 | 3.6 | **251.0** |
| | | 6 | 4.4 | **252.1** |

### [Example 4]

In Example 4, the analysis method of the present embodiment was evaluated using a clinical specimen.

The following specimens were used as the specimen.
(1) Clinical specimen 1: a specimen in which yeast-like fungi and red blood cells are detected in a microscopy
(2) Clinical specimen 2 to 4: a specimen in which yeast-like fungi have been detected by microscopy
(3) Clinical specimen 5 to 10: a specimen in which red blood cells are detected in a microscopy

A urine sample was measured in the same manner as in Example 1, except that the first reagent was prepared by adding 3 g/L chlorphenesin or 6 g/L 2-phenoxyethanol and 2 g/L 1,2-octanediol to the buffer solution described in Example 1. In addition, a urine sample was measured in the same manner as in Example 1 using UF- CELLPACK SF (manufactured by Sysmex) as a first reagent as a control. UF- CELLPACK SF (manufactured by Sysmex) contained 6 g/L of 2-phenoxyethanol. The concentration of the compound contained in the prepared first reagent is shown in the following table. In the following table, the compound of the present embodiment is shown in bold.

**[Table 7]**

| TYPE | ADDED COMPOUND | CONCENTRATION [g/L] | |
|---|---|---|---|
| | | CONCENTRATIO N IN FIRST REAGENT | FINAL CONCENTRATION |
| CONTROL | 2-PHENOXYETHANO L | 6 | 4.4 |
| AROMATIC COMPOUND | **CHLORPHENESIN** | 3 | 2.2 |
| MIXTURE OF ALIPHATIC COMPOUND AND AROMATIC COMPOUND | 2-PHENOXYETHANO L | 6 | 4.4 |
| | | 2 | 1.5 |
| | **1,2-OCTANEDIOL** | | |

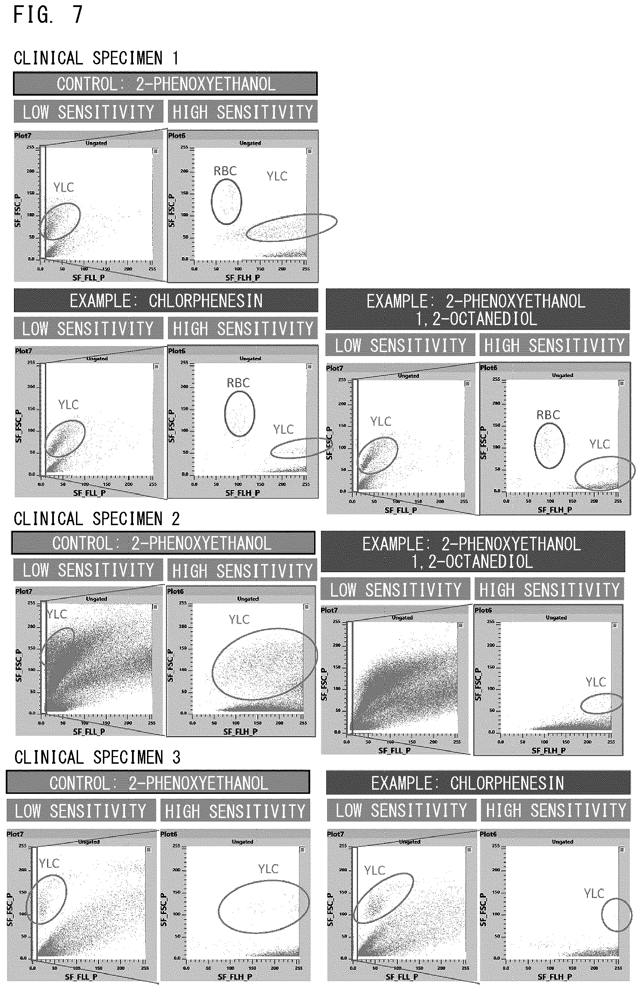

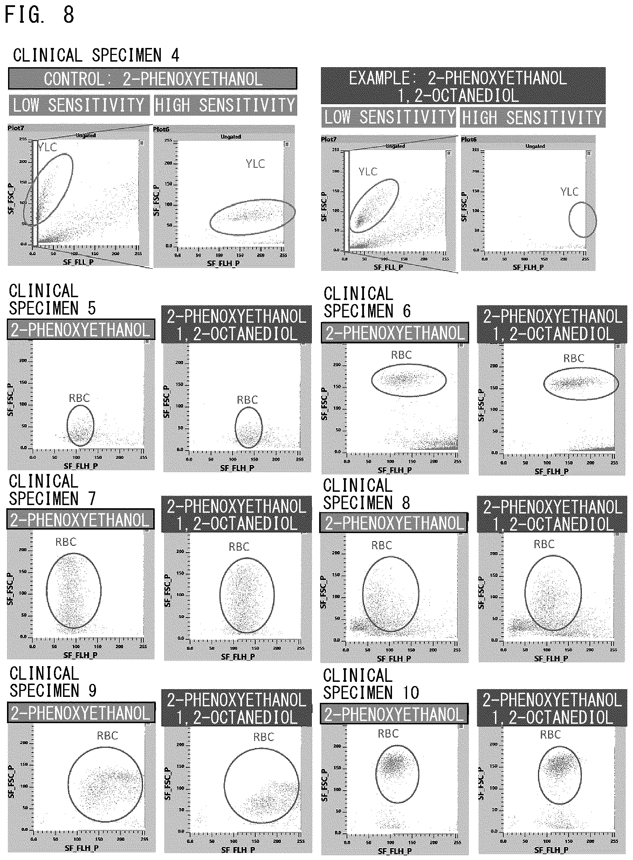

The generated scattergrams are shown in FIGS. 7 to 8. When clinical specimen 1 containing yeast-like fungi and red blood cells was measured, the appearance positions of red blood cell clusters and yeast-like fungal clusters were clearly separated by both reagents. In particular, when 1,2-octanediol or chlorphenesin was used, yeast-like fungi were more strongly stained. Even when clinical specimens 2 to 4 rich in yeast-like fungi were measured, yeast-like fungi were more strongly stained by using 1,2-octanediol or chlorphenesin. Further, from the measurement results of clinical specimens 5 to 10, it was found that the appearance position of red blood cells hardly changed with any reagent.

Further, as can be seen from the scattergram obtained by measuring the clinical specimen 5 to 10 containing red blood cells, the appearance position of the red blood cell cluster on the scattergram is moved by the specimen. This indicates that the red blood cells in the urine vary widely depending on the form (size, shape), the state of the membrane, and the like. In addition, it can be seen that, also in the horizontal axis (fluorescence intensity) direction, staining properties differ depending on the state of the membrane of red blood cells, and appear at various positions. Therefore, according to the analysis method of the present embodiment, it was found that even in a specimen containing yeast-like fungi, since the appearance position of the yeast-like fungal cluster can be greatly shifted to the high value side of the fluorescence intensity, even if the red blood cell count region is set to be wider, yeast-like fungi do not enter and red blood cells in the urine can be detected more accurately.

### [Example 5]

In Example 5, the effect of the osmotic pressure of the reagent on the measurement result was examined.

Each of the following substances was dissolved in purified water to prepare a buffer solution having an osmotic pressure of 200 to 1200 mOsm/kg.

**[Table 8]**

| SUBSTANCE NAME | CONCENTRATION (g/L) |
|---|---|
| TRIS BUFFER (pH 8.0~ 8.2) | 6.1 |
| EDTA-2K | 12.13 |
| SODIUM CHLORIDE | ADJUST OSMOTIC PRESSURE BY AMOUNT ADDED (OSMOTIC PRESSURE 200~1200 mOsm/kg) |

To the buffer solution, the compound shown in the following table was added so as to have a concentration shown in the following table, and a first reagent was prepared.

**[Table 9]**

| ADDED COMPOUND | CONCENTRATION [g/L] | |
|---|---|---|
| | CONCENTRATIO N IN FIRST REAGENT | FINAL CONCENTRATIO N |
| CHLORPHENESIN | 2 | 1.5 |
| 3-PHENOXY-1-PROPANOL | 7 | 5.1 |
| 1,2-OCTANEDIOL | 6 | 4.4 |
| 2-HEPTANOL | 10 | 7.3 |

A urine sample was measured in the same manner as in Example 1, except that the first reagent was prepared as described above. Further, the number of dots appearing in the red blood cell count region was counted, and the number of red blood cells contained per unit amount of the specimen was calculated. The results for the first reagent prepared with each compound are shown in the following table, respectively.

**[Table 10]**

| CHLORPHENESIN | |
|---|---|
| OSMOTIC PRESSURE | RED BLOOD CELL COUNT |
| [mOsm/kg] | [/µL] |
| 200 | 423.0 |
| 298 | 415.5 |
| 391 | 418.9 |
| 494 | 388.3 |
| 600 | 374.3 |
| 683 | 411.2 |
| 902 | 401.0 |
| 1085 | 381.1 |
| 1317 | 410.2 |

**[Table 11]**

| 3-PHENOXY-1-PROPANOL | |
|---|---|
| OSMOTIC PRESSURE | RED BLOOD CELL COUNT |
| [mOsm/kg] | [/µL] |
| 215.0 | 400.6 |
| 299.0 | 392.6 |
| 407.0 | 402.7 |
| 499.0 | 391.6 |
| 593.0 | 409.0 |
| 712.0 | 385.1 |
| 886.0 | 435.7 |
| 1084.0 | 402.3 |
| 1295.0 | 400.7 |

**[Table 12]**

| 1,2-OCTANEDIOL | |
|---|---|
| OSMOTIC PRESSURE | RED BLOOD CELL COUNT |
| [mOsm/kg] | [/µL] |
| 188 | 403.1 |
| 287 | 434.9 |
| 408 | 424.0 |
| 476 | 418.5 |
| 616 | 425.4 |
| 718 | 409.7 |
| 882 | 409.6 |
| 1101 | 418.0 |
| 1297 | 426.0 |

**[Table 13]**

| 2-HEPTANOL | |
|---|---|
| OSMOTIC PRESSURE | RED BLOOD CELL COUNT |
| [mOsm/kg] | [/uL] |
| 186 | 384.7 |
| 296 | 402.2 |
| 407 | 403.0 |
| 484 | 393.2 |
| 604 | 400.1 |
| 694 | 392.8 |
| 895 | 411.2 |
| 1087 | 404.1 |
| 1282 | 388.1 |

From the above results, it was found that, in the range of the tested osmotic pressure of 200 to 1200 mOsm/kg, the measured red blood cell number is not affected by using any compound.

## Claims

1. A method for analyzing a urine sample, comprising:
- obtaining a measurement sample comprising: the urine sample; at least one compound selected from the group consisting of aliphatic compounds and aromatic compounds; and a fluorescent dye that stains nucleic acids or cytoplasm;
- irradiating the measurement sample with light and obtaining fluorescence information of particles contained in the measurement sample; and
- detecting red blood cells based on the fluorescence information,
wherein the aliphatic compound is at least one selected from the group consisting of 1,2-alkanediol having 8 to 10 carbon atoms and 2-alkanol having 7 to 9 carbon atoms, and
wherein the aromatic compound is at least one selected from the group consisting of chlorphenesin, 3-phenoxy-1-propanol, 1-(3-methoxyphenoxy)-2-propanol, and orthophenylphenol.

2. The method according to claim 1, further comprising the step of preparing the measurement sample by mixing the urine sample with a first reagent comprising at least one compound selected from the group consisting of the aliphatic compound and the aromatic compound, and a second reagent comprising the fluorescent dye that stains nucleic acids or cytoplasm.

3. The method according to claim 2, wherein the first reagent is a diluent for the urine sample.

4. The method according to claim 1, wherein the fluorescent dye stains nucleic acids.

5. The method according to claim 1, wherein the fluorescence information is fluorescence intensity.

6. The method according to claim 1, further comprising the step of obtaining scattered light information of particles contained in the measurement sample irradiated with light, wherein the step of detecting red blood cells comprises detecting the red blood cells based on the fluorescence information and the scattered light information.

7. The method according to claim 6, wherein the scattered light information is forward scattered light information.

8. The method according to claim 7, wherein the forward scattered light information is forward scattered light intensity.

9. The method according to claim 1, wherein the 1,2-alkanediol having 8 to 10 carbon atoms is at least one selected from the group consisting of 1,2-octanediol and 1,2-decanediol.

10. The method according to claim 1, wherein the 2-alkanol having 7 to 9 carbon atoms is at least one selected from the group consisting of 2-heptanol and 2-octanol.

11. A method for analyzing a urine sample, comprising:
- obtaining a measurement sample comprising: the urine sample; at least one compound selected from the group consisting of aliphatic compound and aromatic compound; and a fluorescent dye that stains nucleic acids or cytoplasm;
- irradiating the measurement sample with light and obtaining fluorescence information of particles contained in the measurement sample; and
- discriminating between red blood cells and yeast-like fungi based on the fluorescence information;
wherein the aliphatic compound is at least one selected from the group consisting of 1,2-alkanediol having 8 to 10 carbon atoms and 2-alkanol having 7 to 9 carbon atoms, and
wherein the aromatic compound is at least one selected from the group consisting of chlorphenesin, 3-phenoxy-1-propanol, 1-(3-methoxyphenoxy)-2-propanol, and orthophenylphenol.

12. Use of a reagent in the method according to any one of claims 1 to 11, comprising at least one compound selected from the group consisting of the aliphatic compound and the aromatic compound.

13. The use according to claim 12, which is a diluent for the urine sample.
